# EUROPEAN PATENT SPECIFICATION

(11) **EP 1 445 312 B1**
(45) Date of publication and mention of the grant of the patent: **26.12.2012**
(21) Application number: 02803555.8
(22) Date of filing: 21.11.2002
(51) Int. Cl.: C12N 15/113, A61K 31/713, C07H 21/00, G01N 33/50

(54) **METHOD OF INHIBITING GENE EXPRESSION**
VERFAHREN ZUR HEMMUNG DER GENEXPRESSION
PROCEDE POUR INHIBER L'EXPRESSION DE GENES

(30) Priority: 21.11.2001 JP 2001355896
(43) Date of publication of application: 11.08.2004
(73) Proprietor: Astellas Pharma Inc., Tokyo 103-8411 (JP); Saigo, Kaoru, Tokyo 168-0063 (JP)
(72) Inventor: TEI, Kumiko, Tokyo 113-0032 (JP); KAJI, Takahide,c/o Mitsubishi Chemical Corporation, Yokohama-shi, Kanagawa 227-8502 (JP); UEDA, Ryu, c/o Mitsubishi Kagaku Institute Life Sc, Machida-shi, Tokyo 194-8511 (JP); SAIGO, Kaoru, Tokyo 168-0063 (JP)
(74) Representative: HOFFMANN EITLE
(86) International application number: PCT/JP2002/012183
(87) International publication number: WO 2003/044188

(56) References cited:
- WO-A-99/32619
- WO-A-03/040366
- WO-A2-02/10374
- ELBASHIR S M ET AL: "Functional anatomy of siRNAs for mediating efficient RNAi in Drosophila melanogaster embryo lysate" EMBO JOURNAL, vol. 20, no. 23, 3 December 2001 (2001-12-03), pages 6877-6888, XP002225998 ISSN: 0261-4189
- ELBASHIR SAYDA M. ET AL.: 'Duplexes of 21-nucleotide RNAs mediate RNA interference in cultured mammalian cells' NATURE vol. 411, May 2001, pages 494 - 498, XP002206451
- HIROAKI TAHARA: 'Shinkaku seibutsu no keru RNAi to PTGS' PROTEIN, NUCLEIC ACID AND ENZYME vol. 46, no. 14, 01 November 2001, pages 2017 - 2024, XP002965212
- ELBASHIR SAYDA M. ET AL.: 'RNA interference is mediated by 21- and 22-nucleotide RNAs' GENES & DEVELOPMENT vol. 15, January 2001, pages 188 - 200, XP002206453
- BOUTLA ALEXANDER ET AL.: 'Short 5'-phosphorylated double-stranded RNAs induce RNA interference in drosophila' CURRENT BIOLOGY vol. 11, 13 November 2001, pages 1776 - 1780, XP002965213
- HIROHIKO HOHJOH: 'RNA interference (RNAi) induction with various types of synthetic oligonucleotide duplexes in cultured human cells' FEBS LETTERS vol. 521, 2002, pages 195 - 199, XP004362164
- BRUMMELKAMP THIJN R. ET AL.: 'A system for stable expression of short interfering RNAs in mammalian cells' SCIENCE vol. 296, April 2002, pages 550 - 553, XP002234902
- JENN-YAH YU ET AL.: 'RNA interference by expression of short-interfering RNAs and hairpin RNAs in mammalian cells' PROC. NATL. ACAD. SCI. USA vol. 99, no. 9, April 2002, pages 6047 - 6052, XP002204654
- TAKASHI MORITA ET AL.: 'Ho'nyu dobutsu no idenshi kenkyu ni katsuyo sareru RNAi' PROTEIN, NUCLEIC ACID AND ENZYME vol. 47, no. 14, November 2002, pages 1939 - 1945, XP002965214

## Description

### Technical Field

The present invention relates to a method for inhibiting expression of a target gene by transfecting a double-stranded polynucleotide comprising DNA and RNA having a substantially identical nucleotide sequence with at least a partial nucleotide sequence of the target gene into a cell.

### Background Art

Methods for inhibiting expression of a target gene in a cell, tissue, or individual organism include a method (hereinafter, sometimes referred to as "RNAi method") wherein a double-stranded RNA is transfected to the cell, tissue, or individual organism to thereby accelerate degradation of mRNA having homology to its sequence and, as a result, to inhibit expression of a gene which is a template of the mRNA (hereinafter, this effect is sometimes referred to as "RNAi effect"). This technique has hitherto been reported to be effective in individuals of plants (Waterhouse, P. M., et al., Proc. Natl. Acad. Sci. USA., 95, 13959-13964 (1998)), trypanosome (Ngo, H, et al., Proc. Natl. Acad. Sci. USA., 95, 14687-14692 (1998)), hydra (Lohmann J. U., at al., Dev. Biol., 214, 211-214 (1999)), planarian (Sanchez Alvarado, et al., Proc. Natl. Acad. Sci. USA., 96, 5049-5054 (1999)), nematode (Fire, A., et al., Nature, 391, 806-811 (1998), Drosophila (Kannerdell, J. R., et al., Cell, 95, 1017-1026 (1998); Misquitta, L., et al., Proc. Natl. Acad. Sci. USA., 96, 1451-1456 (1999)).

Moreover, although the effect has been reported to be limited in vertebrates, it has been reported that use of a double-stranded RNA each having 19 nucleotides adjointed by 2-nucleotide 3' overhangs enables exhibition of the RNAi effect in cultured cells of vertebrates (Elbashir, S., et al., Nature, 411, 494-498 (2001)). In identification of the gene function described below and the screening method of cell lines suitable for useful substance production, superiority of the use of the RNAi method is apparent but there are problems in that RNA is extremely easily degraded by an ribonuclease, especially in a single-stranded state, and in that cost of production is expensive. Therefore, it has been desired to develop a highly stable polynucleotide which can be used in the RNAi method.

### Disclosure of the Invention

An object of the present invention is to provide a method for inhibiting expression of a target gene having a substantially identical nucleotide sequence with a partial nucleotide sequence of the polynucleotide, by introducing into the cell a double-stranded polynucleotide having an enhanced stability owing to incorporation of DNA.

As a result of extensive studies for achieving the above object, the present inventors have found that, in a hamster culture cell, CHO-KI, transfection with a double-stranded polynucleotide of a hybrid of DNA and RNA having a part of the base sequence of a luciferase gene and that with double-stranded polynucleotide of a chimera of DNA and RNA inhibit expression of the luciferase gene in the cell. Thus, we have accomplished the present invention.

Namely, according to the present invention, inventions described in the following (1) to (8) are provided.
(1) A method for inhibiting expression of a target gene in a cell, which comprises introducing into the cell a double-stranded polynucleotide comprising a chimera of DNA and RNA having a substantially identical polynucleotide sequence with at least a partial nucleotide sequence of the target gene wherein the double-stranded polynucleotide consists of 19-25 nucleotides and at least a half region of the double stranded polynucleotide from the 3' side of the antisense strand is RNA.
(2) The method according to (1) above, wherein the double-stranded polynucleotide comprises a self complementary single strand.
(3) An in vitro method for inhibiting expression of two or more target gene in a cell, which comprises
   introducing two or more double-stranded polynucleotides comprising a chimera of DNA and RNA having a substantially identical polynucleotide sequence with at least a partial nucleotide sequence of the target gene into the cell,
   wherein the double-stranded polynucleotides consists of 19 to 25 nucleotides and at least half region of the double-stranded polynucleotides from 3' side of the antisense strand is RNA.
(4) A method of analysing the function of a gene, which comprises analysing a phenotypic change appearing in the cell, or tissue as a result of inhibition of expression of a target gene by the method according to any one of items 1 to 3.
(5) A method of imparting a specific property to a cell, which comprises inhibiting expression of a target gene in the cell using the method according to any one of items 1 to 3.
(6) A method of preparing a double-stranded polynucleotide that inhibits expression of a target gene in a cell, wherein the double-stranded polynucleotide comprises a chimera of DNA and RNA having a substantially identical polynucleotide sequence with at least a partial nucleotide sequence of the target gene, and
   the double-stranded polynucleotide consists of 19 to 25 nucleotides and at least a half region of the double-stranded polynucleotides from 3' side of the antisense strand is RNA.
(7) A pharmaceutical composition comprising a double-stranded polynucleotide comprising a chimera of DNA and RNA having a substantially identical polynucleotide sequence with at least a partial nucleotide sequence of the target gene, wherein the double-stranded polynucleotide consists of 19-25 nucleotides and at least a half region of the double-stranded polynucleotide from 3'side of the antisense strand is RNA.
(8) A composition for use as a medicament, comprising a double-stranded polynucleotide comprising a chimera of DNA and RNA having a substantially identical polynucleotide sequence with at least a partial nucleotide sequence of the target gene,
   wherein the double-stranded polynucleotide consists of 19-25 nucleotides and at least a half region of the double-stranded polynucleotides from 3' side of the antisense strand is RNA.

### Brief Description of the Drawings

Fig. 1 is a drawing illustrating sequences of the sense single strand polynucleotides consisting of 21 nucleotides and the antisense single strand polynucleotides consisting of 21 nucleotides which were used for preparation of double-stranded polynucleotides. In each sequence, the left means 5' terminal and the right means 3' terminal, and it is shown that a bold character part is RNA and an underlined part is DNA.
Fig. 2 is a drawing illustrating inhibition of expression of a *luc* gene in the case that CHO-KI cells are transfected with double-stranded polynucleotides of DNA-BNA hybrid.
Fig. 3 is a drawing illustrating inhibition of expression of a luc gene in the case that S2 cells are trasfected with double-stranded polynucleotides wherein each sense strand is RNA and each antisense strand is DNA-RNA chimera.
Fig. 4 is a drawing illustrating inhibition of expression of a *luc* gene in the case that HeLa cells and HEK293 cells are transfected with double-stranded polynucleotides wherein each antisense strand is RNA and each sense strand is DNA-RNA chimera.
Fig. 5 is a drawing illustrating inhibition of expression of a *luc* gene in the case that CHO-K1 cells are transfected with double-stranded polynucleotides which are DNA-RNA chimera.

### Best Mode for Carrying Out the Invention

The following will describe the present invention in more detail.

### (1) Double-stranded polynucleotide comprising DNA and RNA for use in the RNAi method

The present invention is a method for inhibiting expression of a target gene in a cell, which comprises introducing into the cell a double-stranded polynucleotide comprising a chimer a of DNA and RNA having a substantially identical polynucleotide sequence with at least a partial nucleotide sequence of a target gene, wherein the double-stranded polynucleotide consists of 19-25 nucleotides and at least a half region of the double-stranded polynucleotide from the 3' side of the antisense strand is RNA.

In the present invention, the target gene may be any gene as long as it can produce mRNA and can optionally be translated into a protein in the cell.

Specifically, it may be endogenous to the cell to be introducing into or a transgene. Moreover, it may be a gene located on a chromosome or an extrachromosomal one. Examples of the extrachromosomal one include those derived from pathogens, such as viruses, bacteria, fungi, or protozoa. The function may be known or unknown or the function may be known in a cell of other organism but is unknown in the recipient.

The double-stranded polynucleotide comprising a chimera of DNA and RNA having a substantially identical nucleotide sequence with at least a partial nucleotide sequence of these genes (hereinafter, this is sometimes referred to as "double-stranded polynucleotide") comprises a substantially identical nucleotide sequence with a sequence having 19-25 nucleotides which may be any part of the nucleotide sequence of the target gene. Herein, "substantially identical" means that the sequence has homology of 50% or more, preferably 70% or more, more preferably 80% or more to the sequence of the target gene. The strand length of the nucleotide may be any length of 19-25 nucleotides of the open reading frame (ORF) of the target gene. However, in cells derived from mammals, there is known the presence of a signal transduction system which is activated in response to a double-stranded RNA having a strand length of 30 nucleotides or more. This is called a interferon response (Mareus, P. I. et at., Interferon, 5, 115-180 (1983)). When such a double-stranded RNA introduces into a cell, start of translation of many genes is non-specifically inhibited through PKR (dsRNA-responsive protein kinese: Bass, B. L., Nature, 411, 428-429 (2001)) and at the same time, RNaseL is activated through 2',5' oligoadenylate synthetase (Bass, B.L., Nature, 411, 428-429 (2001)) to cause non-specific degradation of RNA in the cell. Owing to these non-specific reactions, a specific reaction on the target gene is concealed. Therefore, in the case of using a mammal or a cell or tissue derived from said mammal as a recipient, a double-stranded polynucleotide consisting of 19 to 25, preferably 19 to 23, more preferably 19 to 21 nucleotides is used. The double-stranded polynucleotide of the present invention is not necessarily double-stranded over the whole and includes those having 5'- or 3'-overhang, and the overhang terminal may be from 1 to 5 nucleotides, preferably from 1 to 3 nucleotides, more preferably 2 nucleotides. Moreover, the most preferable example includes one having a structure containing 3'-overhang of 2 nucleotides at 3'-terminal of each polynucleotide strand. A double-stranded polynucleotide means a polynucleotide in which a part having complementarity is double-stranded but may be a polynucleotide in which a self complementary single strand polynucleotide is self-annealed. Examples of the self complementary single strand polynucleotide include those having an inverted repeat and the like.

Furthermore, as mixing of DNA and RNA, a hybrid type of a DNA strand and an RNA strand, a chimera type of DNA and RNA, or the like is used. The hybrid of a DNA strand and an RNA strand may be any one as far as it has an activity to inhibit expression of the target gene when a recipient is transfected with it, but preferably, one in which the sense strand is DNA and the antisense strand is RNA is used. Also, the chimera type of DNA and RNA may be any one as far as it has an activity to inhibit expression of the target gene when a recipient is transfected with it.

In order to enhance stability of the double-stranded polynucleotide, it is preferable to contain DNA as much as possible. However, among the chimera type double-stranded polynucleotides of the present invention, it is preferable to suitably determine a sequence which is required to be RNA for inhibiting expression of the target gene within the range where the inhibition of the expression occurs with carrying out analysis of an inhibition degree of expression of the target gene as described below. Thereby, a functional domain of RNA in the RNAi method can be also identified. As a preferred example of the chimera type thus determined, one in which an upstream partial region of the double-stranded polynucleotide is RNA may be mentioned, for example. Herein, the upstream partial region means 5' side of the sense strand and 3' side of the antisense strand. The upstream partial region preferably means a domain of 9 to 13 nucleotides from the terminal of the upstream of the above double-stranded polynucleotide. Moreover, preferred examples of such chimera type double-stranded polynucleotide include a double-stranded polynucleotide having a strand length of 19 to 21 nucleotides in which at least an upstream half region of the polynucleotide is RNA and the other is DNA. Furthermore, in such double-stranded polynucleotide, an effect of inhibiting expression of the target gene is much higher when the entire antisense strand is RNA.

The method of preparing the double-stranded polynucleotide is not particularly limited but it is preferable to use a chemical synthetic method known per se. In the chemical synthesis, complementary single-stranded polynucleotides are separately synthesized and these are annealed each other by an appropriate method, whereby a double-stranded one can be obtained. A specific example of the method for the annealing include a method wherein the synthesized single-stranded polynucleotides are mixed in a molar ratio of preferably at least about 3:7, more preferably about 4:6, and most preferably substantially equimolar amount (i.e., a molar ratio of about 5:5) and heated to a temperature at which double-stranded one is dissociated and then the whole is gradually cooled. The annealed double-stranded polynucleotide is purified by a usually employed method known per se, if necessary. Example of usable purification methods include a method wherein confirmation with agarose gel is conducted and an remaining single-stranded polynucleotide is optionally removed by, for example, degradation with an appropriate enzyme.

Moreover, in the case that a single-stranded polynucleotide having an inverted repeat is prepared as the single-stranded polynucleotide having self complementarity, the polynucleotide is prepared by a method of chemical synthesis or the like and then a self complementary sequence is annealed in the same manner as above.

### (2) Transfection of cell with double-stranded polynucleotide, and inhibition of expression of target gene

The recipient for transfection with the double-stranded polynucleotide thus prepared may be any one an far as the target gene can be transcribed into RNA or translated into a protein in the cell. Specifically, the recipient for use in the present invention means a **cell.**

The cell for use in the present invention may be any one from the germ line or somatic cell, totipotent or pluripotent cell, dividing or non-dividing cell, parenchyma or epithelium cell, immortalized or transformed cell, or the like. Specifically, the cell may be undifferentiated cells such as a stem cell, cells derived from organs or tissues, or differentiated cells thereof. Moreover, examples of the above differentiated cells include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands. As a specific example of such cells, CHO-KI cell (RIKEN Cell bank), Drosophila S2 cell (Schneider, I., et al., J. Embryol. Exp. Morph., 27, 353-365 (1972)), human HeLa cell (ATCC:CCL-2), human HEK293 cell (ATCC: CRL-1573), or the like is preferably used.

Furthermore, the individual organism to be used as a recipient in the present disclosure specifically include those belonging to plant, animal, protozoan, bacterium, virus, or fungus. The plant may be a monocot, dicot or gymnosperm; the animal may be a vertebrate or invertebrate. Preferred microbes as recipients of the present invention are those used in agriculture or by industry, and those that are pathogenic for plants or animals. Fungi include organisms in both the mold and yeast morphologies. Examples of vertebrate animals include fishes and mammals, such as cattle, goat, pig, sheep, hamster, mouse, rat, monkey, and human; invertebrate animals include nematodes and other worms, Drosophila, and other insects.

As a method of transfecting the double-stranded polynucleotide into a recipient, a calcium phosphate method, an electroporation method, a lipofection method, a viral infection, an immersion in a double-stranded polynucleotide solution, a transformation method, or the like is used, in the case that the recipient is a cell or a tissue.

The quantity of the double-stranded polynucleotide for transfection may be suitably selected depending on the recipient and the target gene, but it is preferable to transfect the polynucleotide in an amount sufficient to transfect at least one copy per cell. Specifically, in the case that the recipient is a human cultured cell and the double-stranded polynucleotide is transfected by a calcium phosphate method, for example, the amount is preferably 0.1 to 1000 nM.

Herein, two or more kinds of the double-stranded polynucleotides may be also transfected simultaneously. In this case, inhibition of expression of two or more target genes is expected in the cell transfected with the polynucleotides (hereinafter, this is referred to as "transfected recipient").

In the present invention, "inhibition of expression of the target gene" means not only complete inhibition of the expression but also inhibition of 20% or more as an expressed amount of mRNA or a protein. The inhibition degree of expression of the target gene can be measured by comparing accumulation of RNA of the target gene or produced amounts of the protein encoded by the target gene in the double-stranded polynucleotide-transfected recipient and non-transfected recipient. The amount of mRNA can be measured by a usually employed method known per se. Specifically, it is conducted by the Northern hybridization analysis, the quantitative reverse transcription PCR, in situ hybridization, or the like. Moreover, the produced amount of the protein can be measured by the Western blot analysis or by determining enzymatic activity of the protein encoded by the target gene.

### (3) Method of analyzing gene function by inhibiting expression of gene in transfected recipient

By analyzing phenotypic change appearing in the transfected recipient as a result of the inhibition of expression of a gene in the transfected recipient by the double-stranded polynucleotide according to the present invention, it is possible to identify function of the gene targeted by the transfected double-stranded polynucleotide.

Herein, the target gene may be a gene whose function is known or a gene whose function in the recipient is unknown. The double-stranded polynucleotide corresponding to the target gene is prepared as described in (1) above and is transfected into the recipient described in (2) in a similar manner. The phenotype whose change is to be analyzed in the transfected recipient is not particularly limited and examples thereof include performances of an organism, such as morphology of the transfected recipient, an amount of a substance in the transfected recipient, an amount of a substance secreted by the transfected recipient, dynamic behavior of a substance in the transfected recipient, adhesion between the transfected recipients, mobility of the transfected recipient, or life of the transfected recipient. In the case that the function of the target gene is known in the other recipient, it is preferable to analyze a phenotype related to the function. As a means for analyzing the phenotypic change, in the case of analyzing morphological change of the transfected recipient, it is possible to use a method of microscopic or visual detection. Moreover, in the case of mRNA as a substance in the transfected recipient, methods of analyzing its amount include Northern hybridization, quantitative reverse transcription PCR, in situ hybridization, or the like. In the case of a protein, methods of analyzing the amount include the Western blot analysis with an antibody using a protein encoded by the target gene as an antigen, a method of measuring enzymatic activity of the protein encoded by the target gene. Since phenotypic change appearing only in the transfected recipient thus analyzed occurs as a result of the inhibition of expression of the target gene, this can be identified as a function of the target gene.

### (4) Method of imparting specific property to cell by inhibiting expression of target gene using double-stranded polynucleotide

By inhibiting expression of the target gene using the double-stranded polynucleotide of the present invention, a specific property can be imparted to a cell. The specific property means a property appearing in the transfected recipient as a result of the inhibition of expression of the target gene. The target gene herein may be a gene wherein a property imparted to the transfected recipient by the inhibition of its expression is already clarified or the function thereof or the function in the transfected recipient is unknown. With regard to the target gene whose function is unknown, by selecting a desired phenotype among the phenotypes exhibited by the transfected recipient after the transfection of the double-stranded polynucleotide thereto, a desired property can be imparted to the transfected recipient.

Specific examples of the desired property to be imparted to the transfected recipient include an intracellular productive function, a function of inhibiting extracellular secretion, a repairing function of an injury of a cell or DNA, a resistant function to a specific disease, and the like. Specifically, in the case that the transfected recipient is a plant individual or the like, the target gene includes genes associated with enzymes relating to fruit ripening, plant structural proteins, pathogenicity, or the like.

The case that the inhibition of expression of the target gene has a resistant function to a specific disease is a case that increase of expression of a specific protein becomes a cause of the specific disease and the target gene includes a gene encoding the above protein, a gene encoding a protein having a function of controlling expression of the above protein, and the like. As a specific example, the target gene is a gene necessary for retention of a carcinogenic/tumorigenic phenotype and the recipient is a cancerous cell, a tumor tissue, or the like.

Since the double-stranded polynucleotide toward such target gene inhibits expression of the protein encoded by the target gene, the polynucleotide can be used as an agent for treating or preventing diseases associated with the target gene. In the case that the double-stranded polynucleotide is used as an active ingredient of the above pharmaceutical agent, the polynucleotide may be used solely but may also be used as a pharmaceutical composition formulated with a pharmaceutically acceptable carrier. The ratio of the active ingredient relative to the carrier at this time may vary between 1 to 90% by weight. Moreover, such pharmaceutical agent can be administered in various forms and these administration forms include oral administration with tablets, capsules, granules, powders, syrups, or the like, or parenteral administration with injections, drips, liposomes, suppositories, or the like. In addition, its dose can be suitably selected depending on symptom, age, body weight, and the like.

The transfected recipient with the double-stranded polynucleotide targeting such a gene is selected depending on a phenotype predicted to be associated with the inhibition of expression of the gene. Moreover, in the double-stranded polynucleotide for transfection, when a sequence encoding a specific genetic marker, e.g., a fluorescent protein, is connected, the selection is possible based on an inhibition degree of expression of the fluorescent protein transfected together with the double-stranded polynucleotide into the recipient. Of these, in the case that a gene functioning for cancer suppression is used as a target gene, cell characters to be selected include characters of malignant tumor, such as increase of growth ability, decrease of cell adhesion ability, or increase of motile (metastatic) ability, and the like. Moreover, in the case that a gene controlling biological rhythm is used as a target gene, cell characters to be selected include disappearance of circadian rhythm and the like. Furthermore, in the case that a gene involved in repair of DNA injury induced by an environmental mutagen is used as a target gene, cell characters to be selected include exhibition of sensitivity toward the mutagen, and the like.

The selected transfected recipient can be established and obtained as a line by a cloning technology known per se, which is suitable to each recipient. Specifically, in the case that the recipient is a cell, the transfected recipient can be established and obtained as a cell line by a limiting dilution method, a method with a drug-resistant marker, or the like, which are cell line-establishing methods in usual cultured cells. The transfected recipient which is obtainable in the present invention and to which a specific function is imparted can be used as a cell line having an increased efficiency of producing or secreting a useful substance, a cell line exhibiting a high sensitivity to an environmental factor providing an injury against cells, DNA, or the like, or a model for treating a disease, which exhibits a character associated with the disease.

Of these, the method of obtaining a cell line to be a model for treating a disease will be described as a further specific applied example of the present disclosure. As the target gene, a gene whose decrease in expressed amount or whose deficiency becomes a cause of a disease may be mentioned. Specifically, there may be mentioned PS1 gene in Alzheimer's disease, XPA/XPD/XPF/XPG genes and DNA polymerase η gene in xeroderma pigmentosum syndrome, APC gene in colon cancer, BRCA1/BRCA2 genes in breast cancer, INS/INSR genes in diabetes, and the like.

By transfecting, for example, a human-derived cultured cell with a double-stranded polynucleotide comprising DNA and RNA having a substantially identical nucleotide sequence with at least a partial nucleotide sequence of these human genes, a human disease model cell can be obtained.

Furthermore, by bringing a test substance into contact with the cell to which the specific property is imparted and by analyzing whether symptom of the disease associated with the gene or change in character appears or not, it is also possible to screen an agent for treating and/or preventing the above disease.

In the case that the substance selected by such a screening is used as an active ingredient of the above pharmaceutical agent, the substance can be used solely but can be also used as a pharmaceutical composition formulated with a **pharmaceutically** acceptable carrier. The ratio of the active ingredient relative to the carrier at this time may vary between 1 to 90% by weight. Moreover, such a pharmaceutical agent can be administered in various forms and these administration forms include oral administration with tablets, capsules, granules, powders, syrups, or the like, or parenteral administration with injections, drips, liposomes, suppositories, or the like. In addition, its dose can be suitably selected depending on symptom, age, body weight, and the like.

### (5) Method of use of primary selection using inhibition degree of expression of indicator gene as index

The methods of the present invention described in the above (1) to (4) are methods of transfecting a recipient with a double-stranded polynucleotide comprising DNA and RNA having a substantially identical nucleotide sequence with at least a partial nucleotide sequence of a target gene. However, in a method of the present disclosure, by further transfecting (a) an expression vector containing DNA encoding an indicator protein, (b) a double-stranded polynucleotide comprising DNA and RNA having a substantially identical nucleotide sequence with at least a partial nucleotide sequence encoding the indicator protein, and primarily screening the transfected recipient using a quantity of signal generated from the indicator protein as a measure, only a transfected recipient in which expression of the gene in the transfected recipient is inhibited can be analyzed, so that an efficient analysis can be conducted.

As a further specific example of the present disclosure, the following will describe a case that a cultured cell derived from a vertebrate animal is used as a recipient and a fluorescent protein is used as an indicator protein. A cultured cell derived from a vertebrate animal is transfected with an expression vector comprising DNA encoding a fluorescent protein, and the cell is cultured, followed by selection of a cell having a quantity of signal generated from the indicator protein of a specific strength or more. The cell selected herein is further transfected with a double-stranded polynucleotide comprising DNA and RNA having a substantially identical nucleotide sequence with at least a partial **nucleotide** sequence of DNA encoding the indicator protein, and the cell is cultured, followed by analysis of an inhibition degree of expression of the indicator gene based on a degree of attenuation of the fluorescence generated from the indicator protein.

Since such each primary screening confirms the transfection of the recipient with the double-stranded and occurrence of the inhibition of expression of the target gene in the transfected recipient, the indicator protein should be a protein which shows correlation between the amount of the protein and the quantity of signal generated therefrom. Specific examples of such a protein include luciferase protein.

Furthermore, in the case of measuring the inhibition degree of expression of the target gene, it is also possible to calculate the amount of the protein encoded by the target gene based on the expressed amount of the indicator protein.

### (6) Kit for use in the present disclosure

The kit for conducting the methods described in (1) to (5) above contains a double-stranded polynucleotide, a vector comprising DNA encoding an indicator protein, a double-stranded polynucleotide comprising DNA and RNA having a substantially identical nucleotide sequence with at least a partial **nucleotide** sequence of the indicator gene, reagents such as enzymes and buffers, reagents for transfecting polynucleotides, and the like. It is not necessary for the kit of the present disclosure to contain all these reagents and the kit may contain any combination of the reagents as far as it is a kit capable of use in the above methods of the present invention.

### Examples

The following will describe the present invention more specifically with reference to Examples but the following Examples should be construed as a help for obtaining a specific recognition of the present invention and hence the scope of the present invention is by no means limited by the following Examples.

### Example 1 Inhibition of expression of target gene by double-stranded DNA-RNA hybrid transfected into CHO-KI cell

### (1) Preparation of DNA-RNA hybrid type double-stranded polynucleotide

A luciferase gene of *Photinus pyralis* (*P. pyralis luc* gene: accession number: U47296) was used as a target gene and pGL3-Control vector (manufactured by Promega) was used as an expression vector comprising the same. The gene fragment of *P. pyralis luc* gene lies between a promoter of SV40 and poly A signal in the vector. A luciferase gene of *Renilla* reniformis was used as an indicator gene and pRL-TK (manufactured by Promega) was used as an expression vector comprising the same.

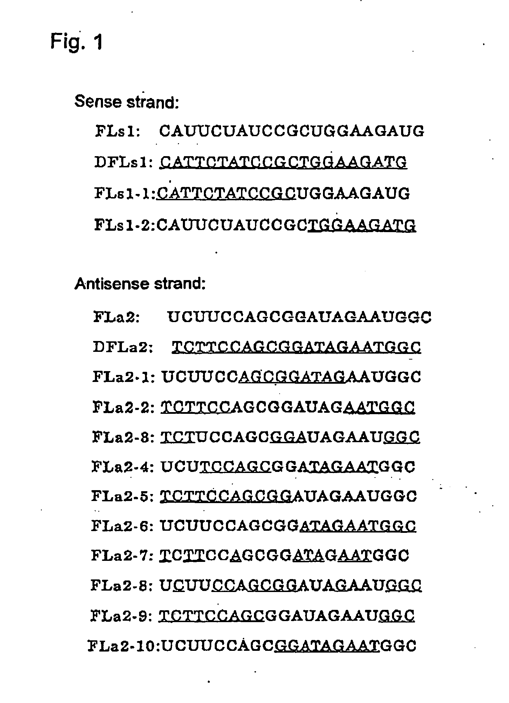

The sense strand of 21 nucleotides for use in the preparation of the double-stranded polynucleotide used in the present Example is represented by SEQ ID NO: 1 (DNA) or SEQ ID NO: 2 (KNA). Moreover, the antisense strand is represented by SEQ ID NO: 3 (DNA) or SEQ ID NO: 4 (RNA). With regard to these sequences, a chimera type single-stranded polynucleotide of DNA or RNA was prepared as shown in Fig. 1. Synthesis of these polynucleotides was entrusted to Genset K.K. via Hitachi Instruments Service Co., Ltd. The sequence of the sense strand polynucleotide corresponds to 38th to 58th nucleotides of the *P. pyralis luc* gene (total length of 1,653 base pairs), which is a target gene in pGL3-Control vector.

The double-stranded RNA, double-stranded DNA and double-stranded DNA-RNA hybrid used for inhibiting expression of the *P. pyralis luc* gene were prepared by annealing the sense strand FLs1 (RNA) or DFLs1 (DNA) with the antisense strand Fla2 (RNA) or DFLa2 (DNA). The annealing was conducted by heating the sense single-stranded polynucleotide and the antisense single-stranded polynucleotide in a 10 mM Tris-HCl (pH 7.5) and 20 mM NaCl reaction liquid at 90°C for 2 minutes and further incubating the whole at 37°C for 1 hour, followed by allowing it to stand until it was cooled to room temperature. Thereby, the sense strand and the antisense strand were annealed to form a double-stranded polynucleotide. The formation of the double-stranded polynucleotide was assayed by an electrophoresis on 2% agarose gel in TBE buffer. Under the above conditions, almost every single-stranded polynucleotide was annealed into a double-stranded polynucleotide.

### (2) Transfection of target gene, indicator gene, and double-stranded polynucleotide into cultured cell

CHO-KI cells (RIKEN Cell Bank) were used as a cultured cell and **Dulbecco's** modified Eagle's medium (manufactured by Gibco BRL) supplemented with inactivated 10% bovine fetal serum (manufactured by Mitsubishi Kasei Corporation) and 10 units/ml penicillin (manufactured by Meiji) and 50 **µg/ml** streptomycin (manufactured by Meiji) as antibiotics was used as a medium. The cells were cultured at 37°C in the presence of 5% CO₂.

The CHO-KI cells were spread on a 24-well plate at a concentration of 0.3 x 10⁶ cells/ml. After 1 day, 1.0 µg pGL3-Control DNA, 0.5 µg pRL-TK DNA, and 0.01, 0.1, 1, 10, or 100 nM of each double-stranded polynucleotide were transfected by Ca-phosphate precipitation method (Saibo Kogaku Handbook, edited by Toshio Kuroki et al., Youdosha (1992)).

### (3) Measurement of gene expression in cultured cell

The cells prepared in Example 1 (2) above were collected after 20 hours and expressed amounts (luciferase activity) of two kinds of luciferase (*Photinus pyralis luc* and *Renilla reniformis luc*) proteins were measured using Dual-Luciferase Reporter Assay System (manufactured by Promega). The measurement of fluorescence was conducted using a Lumat LB9507 luminometer (EG&G Berthold).

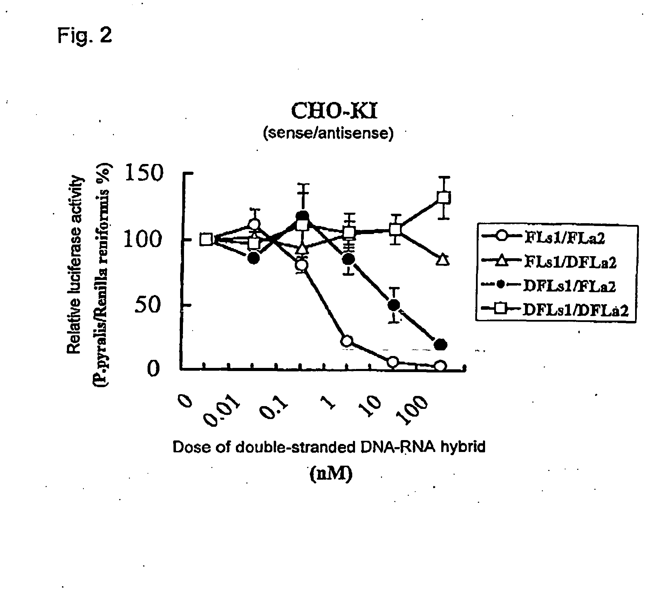

The expression of the gene transfected into CHO-KI cells was inhibited by the DNA-RNA hybrid type double-stranded polynucleotides (Fig. 2). All the values show relative activity of target gene products toward expressed amounts of the indicator gene (enzymatic activity of luciferase). These show the average values of three-time experiments and vertical bars in the figure represent standard deviations. As compared with control groups in which no double-stranded polynucleotide was transfected, 96% inhibition of the expression of the target gene was observed in the case of double-stranded RNA transfected groups when the double-stranded polynucleotide was added in an amount of 100 nM and 80% inhibition was observed in the group in which the double-stranded polynucleotide having a sense strand DNA and an antisense strand RNA was transfected. That is, even when the sense side of the double-stranded polynucleotide was DNA, it was proved that the gene expression can be inhibited in CHO-KI cells when the antisense side was RNA, although the effect was weak as compared with the double-stranded RNA.

### Example 2 Inhibition of expression of target gene by DNA-RNA chimera type double-stranded polynucleotide transfected into Drosophila S2 cell

### (1) Preparation of DNA-RNA chimera type double-stranded polynucleotide

A luciferase gene of *Photinus pyralis* (*P. pyralis luc* gene: accession number: U47296) was used as a target gene. Moreover, a luciferase gene of *Renilla reniformis* was used as an indicator gene. Furthermore, the expression vectors described in Example 1 were also used as expression vectors.

The sense strand of 21 nucleotides for use in the preparation of the double-stranded polynucleotide used in the present Example is represented by SEQ ID NO: 1 (DNA) or SEQ ID NO: 2 (RNA). Moreover, antisense strand is represented by SEQ ID NO: 3 (DNA) or SEQ ID NO: 4 (RNA). With regard to these sequences, chimera type single-stranded polynucleotides of DNA or RNA were prepared as shown in Fig. 1. Synthesis of these polynucleotides was entrusted to Genset K.K. via Hitachi Instruments Service Co., Ltd.

The DNA-RNA chimera type double-stranded polynucleotides used for inhibiting expression of the P. *pyralis luc* gene were prepared by annealing the sense strand FLs1 (single-stranded RNA) with antisense strands Fla2-1, Fla2-2, Fla2-3, Fla2-4, Fla2-5, Fla2-6, Fla2-7, Fla2-8, Fla2-9, and Fla2-10 (DNA-RNA chimera type single-stranded polynucleotides), respectively. The annealing was conducted by reacting the sense single-stranded RNA and the antisense DNA-RNA-chimera type single-stranded polynucleotide in a similar manner to Example 1. The production of the double-stranded polynucleotide was assayed by an electrophoresis on 2% agarose gel in TBE buffer.

### (2) Transfection of target gene, indicator gene, and DNA-RNA chimera type double-stranded polynucleotide into cultured cell

Drosophila S2 cells (Schneider, I., et al., J. Embryol. Exp. Morph., 27, 353-365 (1972)) were used as a cultured cell and Schneider's modified Eagle's medium (manufactured by Gibco BRL) supplemented with inactivated 10% bovine fetal serum (manufactured by Mitsubishi Kasei Corporation) and 10 units/ml penicillin (manufactured by Meiji) and 50 µg/ml streptomycin (manufactured by Meiji) as antibiotics was used as a medium. The cells were cultured at 25°C in the presence of 5% CO₂.

The S2 cells were spread on a 24-well plate at a concentration of 1.0 x 10⁶ cells/ml. After 1 day, 1.0 µg pGL3-Control DNA; 0.05 µg pRL-TK DNA, and 100 nM of each double-stranded polynucleotide were transfected by Ca-phosphate precipitation method (Saibo Kogaku Handbook, edited by Toshio Kuroki et al., Youdosha (1992)).

### (3) Measurement of gene expression in cultured cell

The cells prepared in the Example 2 (2) above were collected after 20 hours and expressed amounts of two kinds of luciferase proteins were measured using Dual-Luciferase Reporter Assay System (manufactured by Promega). The measurement of fluorescence was conducted using a Lumat LB9507 luminometer (EG&G Berthold).

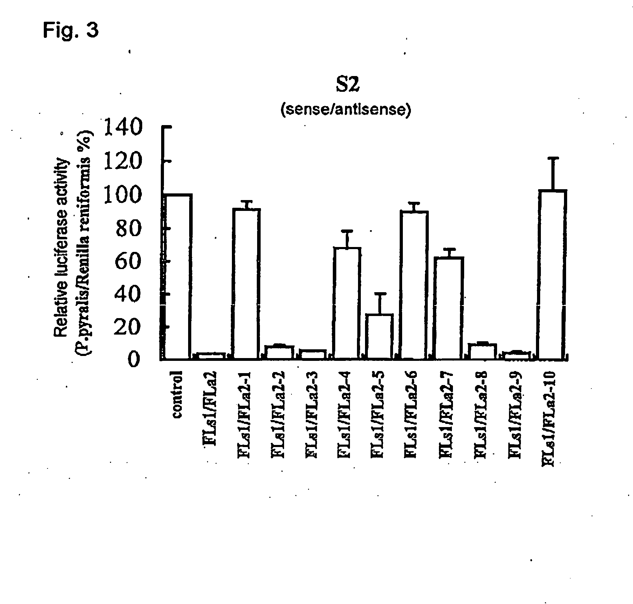

The expression of the gene transfected into S2 cells was inhibited by the DNA-RNA chimera type double-stranded polynucleotides of 21 nucleotides and prepared so that the sense side was fixed to RNA and the antisense side was a chimera type polynucleotide of DNA and RNA (Fig. 3). All the values were determined as relative activity of target gene products toward expressed amounts of the indicator genes (luciferase activity) and the values were shown as average values of three-time experiments and standard deviations. As compared with control groups in which no double-stranded polynucleotide was transfected, the double-stranded polynucleotide with the antisense side Fla2, Fla2-2, Fla2-3, Fla2-8, or Fla2-9 strongly inhibited the gene expression to an approximately equal degree of 96%, 92%, 94%, 91%, or 96%, respectively. The polynucleotide having the antisense side of Fla2-5 exhibited a relatively strong inhibitory effect of 73%. The polynucleotides having the antisense side of Fla2-1, Fla2-4, Fla2-6, Fla2-7, or Fla2-10 exhibited no inhibitory effect or extremely weak effect even when the inhibition was observed. One characteristic common to the double-stranded polynucleotides exhibiting a strong effect is that two nucleotides at 13th and 14th positions from 5' terminal on the sequence of the antisense strand are RNA (UA). On the other hand, in the double-stranded polynucleotide exhibiting a weak effect, both of the corresponding two nucleotides on the sequence of the antisense strand were DNA (TA). Therefore, in the present Example using S2 cells, it is suggested that these two nucleotides are a domain necessary and sufficient for exhibiting a strong RNAi effect.

In the present Example, the RNAi effect was observed even when transfected with a double-stranded polynucleotide in which the part containing this domain on the antisense sequence was reserved as RNA and the other part was substituted with DNA. The double-stranded polynucleotides of 21 nucleotides and prepared by identifying or predicting a domain necessary and sufficient for exhibition of the RNAi effect according to the method as used in the present Example and the other technique, reserving a part containing this domain as RNA, and substituting the other part with DNA are considered to be able to inhibit expression of the target gene by the RNAi effect.

### Example 3 Inhibition of gene expression by DNA-RNA chimera double-stranded polynucleotide transfected into human HeLa cell and human HEK293 cell

### (1) Preparation of DNA-RNA chimera type double-stranded polynucleotide

*P. pyralis luc* gene was used as a target gene and pGL3-Control vector (manufactured by Promega) was used as an expression vector containing the same. Moreover, a *luc* gene of ***Renilla** reniformis* was used as an indicator gene and pRL-TK (manufactured by Promega) was used as an expression vector containing the same.

The sense strand of 21 nucleotides for use in the preparation of the double-stranded polynucleotide used in the present Example is represented by SEQ ID NO: 1 (DNA) or SEQ ID NO. 2 (RNA). Moreover, the antisense strand is represented by SEQ ID NO: 3 (DNA) or SEQ ID NO: 4 (RNA). With regard to these sequences, chimera type single-stranded polynucleotides of DNA or RNA were prepared as shown in Fig. 1. Synthesis of these polynucleotides was entrusted to Genset K.K. via Hitachi Instruments Service, Co., Ltd. The sequence of the sense strand polynucleotide corresponds to 38th to 58th nucleotides of the *P. pyralis luc* gene (total length of 1,653 base pairs), which is a target gene in pGL3-Control vector.

The DNA-RNA chimera type double-stranded polynucleotides used for inhibiting expression of the *P. pyralis luc* gene were prepared by annealing the sense strand FLs1-1 or FLs1-2 (DNA-RNA chimera type single-stranded polynucleotide) with the antisense strand Fla2 (single-stranded RNA). The annealing was conducted by reacting the sense DNA-RNA-chimera type single-stranded polynucleotide and the antisense single-stranded RNA in a similar manner to Example 1. The production of the double-stranded polynucleotide was assayed by an electrophoresis on 2% agarose gel in TBE buffer.

### (2) Transfection of target gene, indicator gene, and DNA-RNA-chimera type double-stranded polynucleotide into cultured cell

pGL3-Control described in (1) above was used as a recombinant expression vector for expressing the target gene and pRL-TK described in (1) above was used as an expression vector of the indicator gene. Human HeLa cells (ATCC: CCL-2) and human HEK293 cells (ATCC: CRL-1573) were used as cultured cells and Dulbecco's modified Eagle's medium (manufactured by Gibco BRL) supplemented with inactivated 10% bovine fetal serum (manufactured by Mitsubishi Kasei Corporation) and 10 units/ml penicillin (manufactured by Meiji) and 50 **µg/ml** streptomycin (manufactured by Meiji) as antibiotics was used as a medium. The cells were cultured at 37°C in the presence of 5% CO₂.

The HeLa cells and HEK293 cells were spread on a 24-well plate at a concentration of 0.5 x 10⁶ cells/ml and 0.25 x **10⁶ cells/ml,-** respectively. After 1 day, 1.0 **µg** pGL3-Control DNA, 1.0 **µg pRL-TK** DNA, and 100 nM of each DNA-RNA chimera type double-stranded polynucleotide were transfected by Ca-phosphate precipitation method.

### (3) Measurement of gene expression in cultured cell

As in Example 1, the cells prepared in Example 3 (2) above were collected after 20 hours and expressed amounts of two kinds of luciferase proteins were measured using Dual-Luciferase Reporter Assay System. The measurement of fluorescence was conducted using a Lumat LB9507 luminometer.

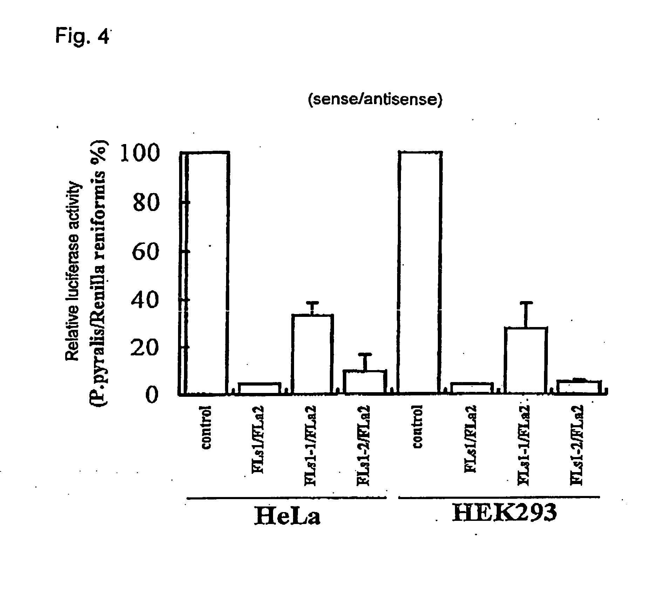

The expression of the gene transfected into HeLa cells and HEK293 cells was inhibited by the double-stranded polynucleotides of 21 nucleotides and prepared so that the antisense side of the double strand was fixed to RNA and the sense side was prepared as a chimera polynucleotide of DNA and RNA (Fig. 4). All the values were determined as relative activity of the target gene products toward expressed amounts of the indicator genes (enzymatic activity of luciferase) and the values were shown as average values of three-time experiments and standard deviations. As compared with control groups in which no double-stranded polynucleotide was transfected, the groups in which the polynucleotides having the sense side of FLs1-2 among the double-stranded polynucleotides was transfected exhibited a strong inhibitory effect of 90% (HeLa cells) or 95% (HEK293 cells), which was equal to the case of the groups in which the double-stranded RNA was transfected. In FLs1-2, 12 nucleotides in the 5' region of the sequence are RNA and nucleotides in the other domain are DNA. Therefore, in the present Example using HeLa cells and HEK293 cells, it is suggested that the whole of the 12 nucleotides in the sense strand or a part thereof is a domain necessary and sufficient for exhibition of a strong RNAi effect when the antisense strand is fixed to RNA.

### Example 4 Inhibition of gene expression by DNA-RNA chimera type double-stranded polynucleotide transfected into CHO-K1 cell

### (1) Preparation of DNA-RNA chimera type double-stranded polynucleotide

*P. pyralis luc* gene was used as a target gene and pGL3-Control vector (manufactured by Promega) was used as an expression vector containing the same. Moreover, a *luc* gene of *Renilla reniformis* was used as an indicator gene and pRL-TK (manufactured by Promega) was used as an expression vector comprising the same.

The sequence of the sense strand of 21 nucleotides for use in the preparation of the double-stranded polynucleotide used in the present Example corresponds to polynucleotides of 8th to 28th (8-28), 38th to 58th (38-58), and 1087th to 1107th (1087-1107) of *P. pyralis luc* gene (total length of 1,653 base pairs), which is a target gene in pGL3-Control vector.

With regard to each base sequence, the sense strand of 8-28 is represented by SEQ ID NO: 5 (DNA) or 6 (RNA) and antisense strand thereof is represented by SEQ ID NO: 7 (DNA) or 8 (RNA). The sense strand of 38-58 is one represented by SEQ ID NO: 1 (DNA) or 2 (RNA) and the antisense strand thereof is represented by SEQ ID NO: 3 (DNA) or 4 (RNA). In addition, the sense strand of 1087-1107 is represented by SEQ ID NO: 9 (DNA) or 10 (RNA) and the antisense strand thereof is represented by SEQ ID NO: 11 (DNA) or 12 (RNA).

With regard to these sequences, there were prepared one in which about an upstream half region (10 to 13 nucleotides) is RNA both for sense and antisense (C), one in which about an upstream half region(10 to 13 nucleotides) is DNA both for sense and antisense (D), one in which antisense is RNA and about an upstream half region (10 to 13 nucleotides) of the sense strand is RNA (E), and one in which sense strand is RNA and about an upstream half region(10 to 13 nucleotides) of the antisense strand is DNA (F). Synthesis of these polynucleotides was entrusted to Genset K.K. via Hitachi Instruments Service, Co., Ltd. and, after formation of chimera type polynucleotides, double stranded one was prepared by annealing each of them. The annealing was conducted by reacting the sense and antisense chimera single-stranded polynucleotides in a similar manner to Example 1. The production of the double-stranded polynucleotide was assayed by an electrophoresis on 2% agarose gel in TBE buffer.

### (2) Transfection of target gene, indicator gene, and DNA-RNA chimera type double-stranded polynucleotide into cultured cell

pGL3-Control described in (1) above was used as a recombinant expression vector for expressing the target gene and pRL-TK described in (1) above was used as an expression vector of the indicator gene. CHO-K1 cells (ATCC: CCL-61) were used as a cultured cell and Dulbecco's modified Eagle's medium (manufactured by Gibco BRL) supplemented with inactivated 10% bovine fetal serum (manufactured by Mitsubishi Kasei Corporation) and 10 units/ml penicillin (manufactured by Meiji) and 50 µg/ml streptomycin (manufactured by Meiji) as antibiotics was used as a medium. The cells were cultured at 37°C in the presence of 5% CO₂.

The **CHO-K1· cells** were spread on a 24-well plate at a concentration of 0.3 x 10⁶ cells/ml. After 1 day, 1.0 µg of pRL-TK DNA and 10 nM or 100 nM of each DNA-RNA chimera type double-stranded polynucleotide were transfected by Ca-phosphate precipitation method.

### (3) Measurement of gene expression in cultured cell

As in Example 1, the cells prepared in Example 4 (2) above were collected after 20 hours and expressed amounts of two kinds of luciferase proteins were measured using Dual-Luciferase Reporter Assay System. The measurement of fluorescence was conducted using a Lumat LB9507 luminometer.

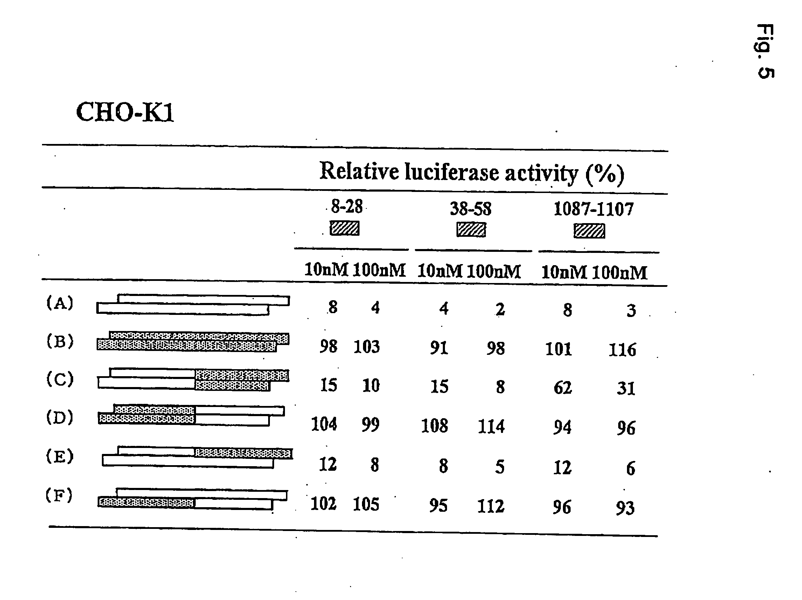

The structures of the double-stranded polynucleotide and the proportion of the luciferase activity of these cells transfected with the polynucleotides to that of a control (a cell in which no double-stranded polynucleotide was transfected) are shown in Fig. 5. In the figure, an open square represents an RNA chain and a filled square represents a DNA chain. As is apparent from the figure, in polynucleotides of any base sequences, the expression of the gene transfected into CHO-K1 cells was inhibited by the double-stranded polynucleotides of 21 nucleotides each in which at least about an upstream half region of the polynucleotide was RNA (Fig. 5 (A), (C) and (E)).

The double-stranded polynucleotides of 21 nucleotides each prepared by identifying or predicting a domain necessary and sufficient for exhibition of the RNAi effect according to the method as used in the present Example and the other technique, reserving a part containing this domain as RNA, and substituting the other part with DNA are considered to be able to inhibit the expression of the target gene by the RNAi effect.

### Industrial Applicability

According to the present invention, using a cultured cell there is provided a means for directly analyzing the functions of genes in human and other organisms. Moreover, according to the disclosure of using an indicator, cells exhibiting an especially high RNAi effect can be primarily screened, so that an effective analysis can be achieved, even among cells exhibiting a weak RNAi effect.

As a conventional inventive technology for use in the same purpose, an RNAi method by transfection with a double-stranded RNA may be mentioned but there are problems in that RNA is extremely easily degraded by a **ribonuclease,** especially in a single-stranded state and cost of the production is expensive. In the present invention, the polynucleotide to be transfected is not limited to be entirely RNA. Therefore, use of a polynucleotide comprising DNA and RNA, specifically a hybrid polynucleotide of a DNA strand and an RNA strand or a DNA-RNA chimera polynucleotide, enables enhancement of stability of the polynucleotide, as substance, for transfection and also reduction of production cost. Therefore, according to the present invention, it is possible to develop the polynucleotide itself as a pharmaceutical preparation for the purpose of treating diseases. Moreover, modifications such as fluorescent labeling, biotin labeling, amination, phosphorylation, and thiolation can be easily conducted over a wide variety with respect to DNA, as compared with the case of RNA. Therefore, when it is used as a pharmaceutical agent or reagent, a function suitable for a purpose can be imparted by conducting such modifications.

## Claims

1. An in vitro method for inhibiting expression of a target gene in a cell, which comprises introducing into the cell a double-stranded polynucleotide comprising a chimera of DNA and RNA having a substantially identical polynucleotide sequence with at least a partial nucleotide sequence of the target gene, wherein the double-stranded polynucleotide consists of 19-25 nucleotides and at least a half region of the double-stranded polynucleotide from the 3' side of the antisense strand is RNA.

2. The method according to claim 1, wherein the double-stranded polynucleotide comprises a self-complementary single strand.

3. An in vitro method for inhibiting expression of two or more target gene in a cell, which comprises
introducing two or more double-stranded polynucleotides comprising a chimera of DNA and RNA having a substantially identical polynucleotide sequence with at least a partial nucleotide sequence of the target gene into the cell,
wherein the double-stranded polynucleotides consists of 19 to 25 nucleotides and at least half region of the double-stranded polynucleotides from 3' side of the antisense strand is RNA.

4. A method of analysing the function of a gene, which comprises analysing a phenotypic change appearing in the cell, or tissue as a result of inhibition of expression of a target gene by the method according to any one of claims 1 to 3.

5. A method of imparting a specific property to a cell, which comprises inhibiting expression of a target gene in the cell using the method according to any one of claims 1 to 3.

6. A method of preparing a double-stranded polynucleotide that inhibits expression of a target gene in a cell, wherein
the double-stranded polynucleotide comprises a chimera of DNA and RNA having a substantially identical polynucleotide sequence with at least a partial nucleotide sequence of the target gene, and
the double-stranded polynucleotide consists of 19 to 25 nucleotides and at least a half region of the double-stranded polynucleotides from 3' side of the antisense strand is RNA.

7. A pharmaceutical composition comprising a double-stranded polynucleotide comprising a chimera of DNA and RNA having a substantially identical polynucleotide sequence with at least a partial nucleotide sequence of the target gene, wherein the double-stranded polynucleotide consists of 19-25 nucleotides and at least a half region of the double-stranded polynucleotide from 3'side of the antisense strand is RNA.

8. A composition for use as a medicament, comprising a double-stranded polynucleotide comprising a chimera of DNA and RNA having a substantially identical polynucleotide sequence with at least a partial nucleotide sequence of the target gene,
wherein the double-stranded polynucleotide consists of 19-25 nucleotides and at least a half region of the double-stranded polynucleotides from 3' side of the antisense strand is RNA.

## Patentansprüche

1. In vitro-Verfahren zum Inhibieren der Expression eines Zielgens in einer Zelle, das das Einbringen eines doppelsträngigen Polynukleotids in eine Zelle umfasst, wobei das Polynukleotid eine Chimäre von DNA und RNA umfasst, die eine im Wesentlichen identische Polynukleotidsequenz mit mindestens einer Teilnukleotidsequenz des Zielgens aufweist, wobei das doppelsträngige Polynukleotid aus 19 bis 25 Nukleotiden besteht und mindestens eine Halbregion des doppelsträngigen Polynukleotids von der 3'-Seite des Antisense-Strangs RNA ist.

2. Verfahren gemäß Anspruch 1, wobei das doppelsträngige Polynukleotid einen selbst-komplementären Einzelstang umfasst.

3. In vitro-Verfahren zum Inhibieren der Expression von zwei oder mehr Zielgenen in einer Zelle, das umfasst
Einbringen von zwei oder mehr doppelsträngigen Polynukleotiden, die eine Chimäre von DNA und RNA mit einer im Wesentlichen identischen Polynukleotidsequenz mit mindestens einer Teilnukleotidsequenz des Zielgens umfassen, in die Zelle,
wobei die doppelsträngigen Polynukleotide aus 19 bis 25 Nukleotiden bestehen und mindestens eine Halbregion des doppelsträngigen Polynukleotids von der 3'-Seite des Antisense-Strangs RNA ist.

4. Verfahren zum Analysieren der Funktion eines Gens, das das Analysieren einer phenotypischen Veränderung umfasst, die in der Zelle oder im Gewebe als Ergebnis der Inhibition der Expression eines Zielgens durch das Verfahren gemäß einem der Ansprüche 1 bis 3 hervorgerufen wird.

5. Verfahren zum Verleihen einer bestimmten Eigenschaft an eine Zelle, das das Inhibieren der Expression eines Zielgens in der Zelle unter Verwendung des Verfahrens gemäß einem der Ansprüche 1 bis 3 umfasst.

6. Verfahren zum Herstellen eines doppelsträngigen Polynukleotids, das die Expression eines Zielgens in einer Zelle inhibiert, wobei
das doppelsträngige Polynukleotid eine Chimäre von DNA und RNA mit im Wesentlichen identischer Polynukleotidsequenz mit mindestens einer Teilnukleotidsequenz des Zielgens umfasst, und
das doppelsträngige Polynukleotid aus 19 bis 25 Nukleotiden besteht und mindestens eine Halbregion des doppelsträngigen Polynukleotids von der 3'-Seite des Antisense-Strangs RNA ist.

7. Pharmazeutische Zusammensetzung, umfassend ein doppelsträngiges Polynukleotid, wobei das Polynukleotid eine Chimäre von DNA und RNA umfasst, die eine im Wesentlichen identische Polynukleotidsequenz mit mindestens einer Teilnukleotidsequenz des Zielgens umfasst,
wobei das doppelsträngige Polynukleotid aus 19 bis 25 Nukleotiden besteht und mindestens eine Halbregion des doppelsträngigen Polynukleotids von der 3'-Seite des Antisense-Strangs RNA ist.

8. Zusammensetzung zur Verwendung als Medikament, umfassend ein doppelsträngiges Polynukleotid, wobei das Polynukleotid eine Chimäre von DNA und RNA umfasst, die eine im Wesentlichen identische Polynukleotidsequenz mit mindestens einer Teilnukleotidsequenz des Zielgens umfasst,
wobei das doppelsträngige Polynukleotid aus 19 bis 25 Nukleotiden besteht und mindestens eine Halbregion des doppelsträngigen Polynukleotids von der 3'-Seite des Antisense-Strangs RNA ist.

## Revendications

1. Procédé in vitro destiné à inhiber l'expression d'un gène cible dans une cellule, qui comprend le fait d'introduire dans la cellule un polynucléotide double brin comprenant une chimère d'ADN et d'ARN ayant une séquence de polynucléotides sensiblement identique à au moins une séquence nucléotidique partielle du gène cible, dans lequel le polynucléotide double brin est constitué de 19-25 nucléotides et au moins une demi-région du polynucléotide double brin à partir du côté 3' du brin antisens est un ARN.

2. Procédé selon la revendication 1, dans lequel le polynucléotide double brin comprend un brin unique auto complémentaire.

3. Procédé in vitro destiné à inhiber l'expression de deux ou plus de gènes cibles dans une cellule, qui comprend le fait
d'introduire de deux ou plus de polynucléotides double brin comprenant une chimère d'ADN et d'ARN ayant une séquence de polynucléotides sensiblement identique avec au moins une séquence nucléotidique partielle du gène cible dans la cellule,
dans lequel les polynucléotides double brin sont constitués de 19 à 25 nucléotides et au moins une demi-région des polynucléotides double brin du côté 3' du brin antisens est un ARN.

4. Procédé d'analyse de la fonction d'un gène, qui comprend l'analyse d'un changement phénotypique qui apparaît dans la cellule, ou dans un tissu en tant qu'un résultat d'une inhibition de l'expression d'un gène cible par le procédé selon l'une quelconque des revendications 1 à 3.

5. Procédé destiné à donner une propriété spécifique à une cellule, qui comprend une inhibition de l'expression d'un gène cible dans la cellule en utilisant le procédé selon l'une quelconque des revendications 1 à 3.

6. Procédé destiné à la préparation d'un polynucléotide double brin qui inhibe une expression d'un gène cible dans une cellule, dans lequel
le polynucléotide double brin comprend une chimère d'ADN et d'ARN ayant une séquence de polynucléotides sensiblement identique à au moins une séquence nucléotidique partielle du gène cible, et
le polynucléotide double brin est constitué de 19 à 25 nucléotides et au moins une demi-région des polynucléotides double brin à partir du côté 3' du brin antisens est un ARN.

7. Composition pharmaceutique comprenant un polynucléotide double brin comprenant une chimère d'ADN et d'ARN ayant une séquence de polynucléotides sensiblement identique avec au moins une séquence nucléotidique partielle du gène cible, dans laquelle le polynucléotide double brin est constitué de 19-25 nucléotides et au moins une demi-région du polynucléotide double brin du côté 3' du brin antisens est un ARN.

8. Composition conçue pour une utilisation en tant que médicament, comprenant un polynucléotide double brin comprenant une chimère d'ADN et d'ARN ayant une séquence de nucléotides sensiblement identique avec au moins une séquence nucléotidique partielle du gène cible,
dans lequel le polynucléotide double brin est constitué de 19-25 nucléotides et au moins une demi-région de polynucléotides double brin du côté 3' du brin antisens est un ARN.


### SEQUENCE LISTING

<110> MITSUBISHI CHEMICAL CORPORATION
<120> Genetic Inhibition by doublestranded polynucleotide
<130> P-43361
<140> JP 2001-355896
   <141> 2001-11-21
<160> 8
<170> PatentIn Ver. 2.0
<210> 1
   <211> 21
   <212> DNA
   <213> Artificial Sequence
<220>
   <223> Description of Artificial Sequence:synthesized
<400> 1
   cattctatcc gctggaagat g 21
<210> 2
   <211> 21
   <212> RNA
   <213> Artificial Sequence
<220>
   <223> Description of Artificial Sequence:synthesized
<400> 2
   cauucuaucc gcuggaagau g 21
<210> 3
   <211> 21
   <212> DNA
   <213> Artificial Sequence
<220>
   <223> Description of Artificial Sequence:synthesized
<400> 3
   tcttccagcg gatagaatgg c 21
<210> 4
   <211> 21
   <212> RNA
   <213> Artificial Sequence
<220>
   <223> Description of Artificial Sequence:synthesized
<400> 4
   ucuuccagcg gauagaaugg c 21
<210> 6
   <211> 21
   <212> DNA
   <213> Artificial Sequence
<220>
   <223> Description of Artificial Sequence:synthesized
<400> 5
   acgccaaaaa cataaagaaa g 21
<210> 6
   <211> 21
   <212> RNA
   <213> Artificial Sequence
<220>
   <223> Description of Artificial Sequence:synthesized
<400> 6
   acgccaaaaa cauaaagaaa g 21
<210> 7
   <211> 21
   <212> DNA
   <213> Artificial Sequence
<220>
   <223> Description of Artificial Sequence:synthesized
<400> 7
   ttctttatgt ttttggcgtc t 21
<210> 8
   <211> 21
   <212> RNA
   <218> Artificial Sequence
<220>
   <223> Description of Artificial Sequence:synthesized
<400> 8
   uucuuuaugu uuuuggcguc u 21
<210> 9
   <211> 21
   <212> DNA
   <213> Artificial Sequence
<220>
   <223> Description of Artificial Sequence:synthesized
<400> 9
   ggtaaagttg ttttattttt t 21
<210> 10
   <211> 21
   <212> RNA
   <213> Artificial Sequence
<220>
   <223> Description of Artificial Sequence:synthesized
<400> 10
   gguaaaguug uuuuauuuuu u 21
<210> 11
   <211> 21
   <212> DNA
   <213> Artificial Sequence
<220>
   <223> Description of Artificial Sequence:synthesized
<400> 11
   aaaatggaac aactttaccg a 21
<210> 12
   <211> 21
   <212> RNA
   <213> Artificial Sequence
<220>
   <223> Description of Artificial Sequence:synthesized
<400> 12
   aaaauggaac aacuuuaccg a 21